# FASCICULE DE BREVET EUROPEEN

(11) **EP 3 345 214 B1**
(45) Date de publication et mention de la délivrance du brevet: **26.05.2021**
(21) Numéro de dépôt: 16766858.1
(22) Date de dépôt: 29.08.2016
(51) Int. Cl.: H01L 21/67, H01L 21/673, G01N 33/00, G01N 21/94

(54) **PROCÉDÉ ET STATION DE MESURE DE LA CONTAMINATION D'UNE BOÎTE DE TRANSPORT POUR LE CONVOYAGE ET LE STOCKAGE ATMOSPHÉRIQUE DE SUBSTRATS**
VERFAHREN UND STATION ZUR MESSUNG DER VERSCHMUTZUNG EINER TRANSPORTBOX ZUR FÖRDERUNG UND ATMOSPHÄRISCHEN LAGERUNG VON SUBSTRATEN
METHOD AND STATION FOR MEASURING THE CONTAMINATION OF A TRANSPORT BOX FOR THE CONVEYING AND ATMOSPHERIC STORAGE OF SUBSTRATES

(30) Priorité: 02.09.2015 FR 1558115
(43) Date de publication de la demande: 11.07.2018
(73) Titulaire: Pfeiffer Vacuum, 74000 Annecy (FR)
(72) Inventeur: LE-BARILLEC, Olivier, 74370 ANNECY (FR); BOUNOUAR, Julien, 74000 Annecy (FR)
(74) Mandataire: Croonenbroek, Thomas Jakob
(86) Numéro de dépôt international: PCT/EP2016/070309
(87) Numéro de publication internationale: WO 2017/037014

(56) Documents cités:
- JP-A- H10 199 847
- KR-B1- 101 271 181

## Description

La présente invention se rapporte à un procédé de mesure de la contamination d'une boîte de transport pour le convoyage et le stockage atmosphérique de substrats, tels que les plaquettes de semi-conducteurs ou les photomasques. L'invention se rapporte également à une station de mesure de la contamination d'une boîte de transport.

Dans l'industrie de fabrication de semi-conducteurs, les boîtes de transport et de stockage atmosphérique de substrats, tels que les plaquettes de semi-conducteurs (ou « wafer » en anglais) ou les photomasques, déterminent un volume confiné à pression atmosphérique, isolé de l'environnement d'utilisation pour le transport et le stockage d'un ou de plusieurs substrats, d'un équipement à l'autre ou pour stocker les substrats entre deux étapes de fabrication.

On distingue notamment les boîtes standardisées de transport et de stockage de plaquettes à ouverture latérale de type FOUP (" Front Opening Unified Pod " en anglais) et FOSB (" Front Opening Shipping Box ") ou à ouverture par le fond de type SMIF Pod (" Standard Mechanical Interface Pod " en anglais), les boîtes dites " open cassette " en anglais et les boîtes standardisées de transport et de stockage de photomasques de type RSP (" Reticle SMIF Pod " en anglais).

Ces boîtes en matériau plastique, tel qu'en polycarbonate, peuvent être polluées par des gaz de procédés de fabrication, tels que les gaz HF, HCl, NH₃, PGMEA, ces gaz étant relâchés notamment par les plaquettes semi-conductrices ayant subi des opérations préalables de fabrication.

Les gaz relâchés peuvent s'adsorber sur les surfaces internes des boîtes, puis diffuser dans le polymère, conduisant à l'accumulation de molécules polluantes dans le polymère. Ces molécules polluantes peuvent désorber ultérieurement, puis s'adsorber sur les substrats stockés dans ces boîtes, et éventuellement réagir chimiquement avec la surface, ce qui peut créer des défauts sur les surfaces des substrats.

L'acide fluorhydrique sous forme gazeuse notamment, est un composé qui impacte particulièrement le rendement de fabrication des puces électroniques. En effet, cette molécule peut générer des défauts sur la plaquette de semi-conducteurs. Ces défauts, généralement sous forme de cristaux, peuvent être à l'origine de dysfonctionnements des puces.

Une méthode utilisée aujourd'hui pour mesurer les espèces gazeuses polluantes potentiellement présentes dans les boîtes de transport, consiste à réaliser un prélèvement par bullage dans de l'eau dé-ionisée. Le prélèvement est analysé par Chromatographie Ionique. Cette opération est assez longue, elle dure en moyenne deux heures, et ne peut être réalisée qu'hors production.

Une autre méthode connue est décrite dans le document EP1703547. On contrôle l'atmosphère intérieure des boîtes de transport par des moyens d'analyse externes performants mis en communication avec l'atmosphère interne de la boîte de transport. Grâce à cette disposition, il est possible de faire une analyse en temps réel, en cours de production, de traces de gaz contaminants contenues dans les boîtes de transport.

Cette mesure de contamination des boîtes de transport de substrats doit être réalisée à cadence élevée afin de ne pas perturber le rendement de production. Toutefois, lorsqu'une espèce contaminante est mesurée en quantité importante dans l'atmosphère intérieure d'une boîte de transport, la ligne de mesure mettant en communication la boîte avec les moyens d'analyse peut être polluée de manière significative. Il est alors nécessaire de dépolluer la ligne préalablement à tout nouveau contrôle de boîte afin d'éviter de fausser une mesure ultérieure.

Cependant, la dépollution de la ligne de mesure après qu'une espèce gazeuse polluante ait été détectée peut être longue. En effet, l'acide fluorhydrique notamment, adhère particulièrement aux parois du fait du caractère polaire de la molécule. Ce phénomène peut donc augmenter le temps d'attente entre deux mesures consécutives pour que la concentration d'acide fluorhydrique retrouve un niveau bas pour lequel l'impact sur la mesure suivante est devenu négligeable. Egalement, au cours de la mesure, l'absorption de l'acide fluorhydrique sur les parois peut empêcher sa détection par les moyens d'analyse.

KR 101 271 181 B1 (WITHTECH INC) décrit un procédé de mesure de la contamination d'une boite de transport dans lequel un flux de gaz est fourni dans le dispositif de mesure, ce flux de gaz ne comportant pas de vapeur d'eau.

Un des buts de la présente invention est de proposer une méthode et une station permettant de réduire le temps d'attente entre deux mesures. Un autre but de la présente invention est de faciliter la mesure des espèces gazeuses contaminantes présentes dans la boîte de transport.

A cet effet, l'invention a pour objet un procédé de mesure de la contamination d'une boîte de transport pour le convoyage et le stockage atmosphérique de substrats dans lequel on mesure la concentration d'au moins une espèce gazeuse à l'intérieur de la boîte de transport par un dispositif de mesure comprenant au moins un analyseur de gaz et une canalisation de mesure reliant ledit au moins un analyseur de gaz à une interface, l'interface mettant en communication la canalisation de mesure avec l'atmosphère interne d'au moins une boîte de transport, caractérisé en ce qu'on fournit un flux de gaz contenant de la vapeur d'eau dans le dispositif de mesure.

Les inventeurs ont en effet constaté que le taux d'humidité du flux de gaz introduit dans le dispositif de mesure, influence la capacité de certaines espèces gazeuses contaminantes, telles que l'acide fluorhydrique, à désorber des parois. Leurs expériences montrent que l'introduction d'un flux de gaz humide permet d'accélérer la désorption de l'espèce gazeuse contaminante.

Selon une ou plusieurs caractéristiques du procédé de mesure, prise seule ou en combinaison,
- au moins une espèce gazeuse dont on mesure la concentration est l'acide fluorhydrique,
- le flux de gaz contenant de la vapeur d'eau présente un taux d'humidité supérieur à 40%, tel que supérieur à 95%,
- le flux de gaz contenant de la vapeur d'eau est de l'air humide,
- on fournit un flux de gaz contenant de la vapeur d'eau dans le dispositif de mesure pendant que l'on réalise une mesure de contamination,
- on fournit un flux de gaz contenant de la vapeur d'eau dans le dispositif de mesure après avoir réalisé une mesure de contamination et isolé l'interface,
- on fournit un flux de gaz contenant de la vapeur d'eau dans le dispositif de mesure après avoir réalisé une mesure de contamination pour laquelle la concentration de l'espèce gazeuse a dépassé un seuil prédéterminé,
- on fournit un flux de gaz contenant de la vapeur d'eau dans le dispositif de mesure après chaque mesure d'espèce gazeuse,
- on fournit un flux de gaz présentant un premier taux d'humidité si la concentration de l'espèce gazeuse est inférieure à un seuil prédéterminé et on fournit un flux de gaz présentant un deuxième taux d'humidité supérieur au premier taux d'humidité si la concentration de l'espèce gazeuse dépasse le seuil prédéterminé.

L'invention a aussi pour objet une station de mesure de la contamination d'une boîte de transport pour le convoyage et le stockage atmosphérique de substrats comprenant:
- un dispositif de mesure comprenant au moins un analyseur de gaz et une canalisation de mesure reliée audit au moins un analyseur de gaz, et
- une interface pour mettre en communication la canalisation de mesure avec l'atmosphère interne d'au moins une boîte de transport de manière à effectuer une analyse d'au moins une espèce gazeuse contenue dans l'atmosphère interne de la boîte de transport par ledit au moins un analyseur de gaz,
caractérisée en ce qu'elle comporte un générateur d'humidité configuré pour fournir un flux de gaz contenant de la vapeur d'eau dans le dispositif de mesure.

Selon une ou plusieurs caractéristiques de la station de mesure prise seule ou en combinaison,
- le générateur d'humidité est raccordé à la canalisation de mesure reliant ledit au moins un analyseur de gaz à l'interface, la station de mesure comportant au moins une vanne agencée sur la canalisation de mesure, la vanne étant configurée pour mettre en communication l'interface avec ledit au moins un analyseur de gaz ou ledit au moins un analyseur de gaz avec le générateur d'humidité,
- ledit au moins un analyseur de gaz comporte une pompe de prélèvement,
- ledit au moins un analyseur de gaz comporte un capteur optique fonctionnant par spectroscopie d'absorption.

D'autres caractéristiques et avantages de l'invention ressortiront de la description suivante, donnée à titre d'exemple, sans caractère limitatif, en regard des dessins annexés sur lesquels:
- la figure 1 représente une vue schématique d'éléments d'une station de mesure de la contamination d'une boîte de transport,
- la figure 2 représente un exemple d'interface de la station de mesure de la figure 1, et
- la figure 3 est un graphique représentant la quantité d'acide fluorhydrique désorbée des parois du dispositif de mesure en fonction du temps et du taux d'humidité du flux de gaz introduit dans le dispositif de mesure.

Sur ces figures, les éléments identiques portent les mêmes numéros de référence.

Les réalisations suivantes sont des exemples. Bien que la description se réfère à un ou plusieurs modes de réalisation, ceci ne signifie pas nécessairement que chaque référence concerne le même mode de réalisation, ou que les caractéristiques s'appliquent seulement à un seul mode de réalisation. De simples caractéristiques de différents modes de réalisation peuvent également être combinées pour fournir d'autres réalisations.

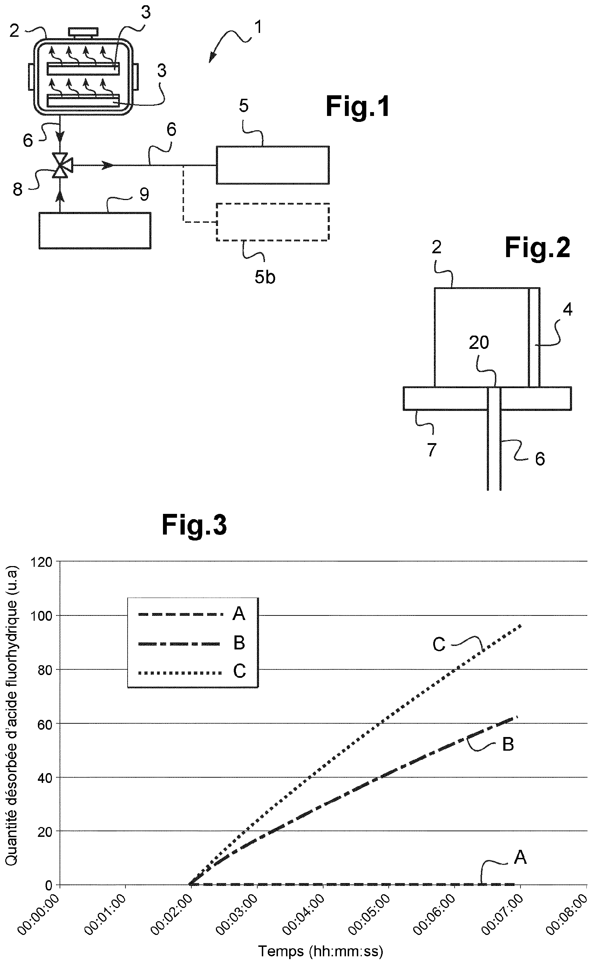

La figure 1 représente une station de mesure 1 de la contamination d'une boîte de transport 2 pour le convoyage et le stockage atmosphérique de substrats 3.

La boîte de transport 2 peut notamment être une boîte de transport standardisée de type FOUP, FOSB, SMIF Pod, RSP ou " Open Cassette ".

On utilise par exemple des boîtes de transport 2 standardisées à ouverture frontale de type FOUP, comme illustré sur la figure 1.

La boîte de transport 2 est en matériau plastique comme par exemple en matériau polycarbonate.

La boîte de transport 2 comporte des parois confinant un volume intérieur destiné au stockage de substrats 3, tels que des plaquettes de semi-conducteurs ou des photomasques.

Comme on l'a représenté de manière schématique sur la figure 2, de telles boîtes de transport 2 de type FOUP comprennent une porte frontale 4 dimensionnée pour l'introduction et l'extraction des substrats 3, et comprennent généralement un ou plusieurs orifices 20 inférieurs munis d'un filtre protégeant les substrats 3 de la contamination particulaire pouvant provenir de l'environnement extérieur à la boîte de transport 2.

La boîte de transport 2 délimite un volume confiné. Cependant, l'air ambiant peut passer à travers les fuites du joint de la porte et à travers les orifices 20, en privilégiant toutefois la fuite par les filtres des orifices 20. Les orifices 20 comportant des filtres permettent l'entrée et la sortie de l'air ambiant, pour équilibrer les pressions notamment lors de la fermeture et de l'ouverture de la porte 4.

La station de mesure 1 comporte un dispositif de mesure et une interface 7.

Le dispositif de mesure comporte au moins un analyseur de gaz 5, 5b et une canalisation de mesure 6 reliée audit au moins un analyseur de gaz 5, 5b.

La canalisation de mesure 6 est par exemple réalisée en perfluoroalkoxy (également appelé PFA) ou en Polytétrafluoroéthylène (également appelé PTFE), pour limiter l'adhérence des espèces gazeuses polluantes aux parois.

L'interface 7 permet de mettre en communication la canalisation de mesure 6 avec l'atmosphère interne d'au moins une boîte de transport 2 de manière à effectuer une analyse d'au moins une espèce gazeuse potentiellement présente dans l'atmosphère interne de la boîte de transport 2, par un ou plusieurs analyseur(s) de gaz 5, 5b.

La boîte de transport 2 est par exemple positionnée sur l'interface 7 au moyen de picots de positionnement normalisés. L'interface 7 comporte en outre des moyens de retenue de la boîte de transport 2, tels que des doigts de verrouillage pivotants.

L'interface 7 assure la mise en connexion de la boîte de transport 2, par son orifice 20, avec la canalisation de mesure 6 qui conduit les gaz jusqu'à l'entrée d'au moins un analyseur de gaz 5, 5b.

Si la boîte de transport ne comporte pas d'orifice 20 dans sa paroi inférieure, l'interface 7 comporte alors des moyens d'ouverture de la porte de la boîte de transport, et des moyens collecteurs assurant un isolement suffisant vis-à-vis de l'atmosphère de la salle blanche. On prévoit alors que la mesure soit prise à l'entrouverture de la porte de la boîte de transport.

La station de mesure 1 comporte également un générateur d'humidité 9.

Le générateur d'humidité 9 est configuré pour fournir un flux de gaz contenant de la vapeur d'eau avec une humidité relative supérieure à 0% et inférieure à 100%, non condensée, dans le dispositif de mesure, c'est-à-dire dans la canalisation de mesure 6 et/ou dans au moins un analyseur de gaz 5, 5b.

Le générateur d'humidité 9 est par exemple raccordé à la canalisation de mesure 6 reliant au moins un analyseur de gaz 5, 5b à l'interface 7.

La station de mesure 1 peut comporter une vanne trois voies 8 agencée sur la canalisation de mesure 6. La vanne trois voies 8 est d'une part, configurée pour mettre en communication l'interface 7 avec au moins un analyseur de gaz 5, 5b le générateur d'humidité 9 étant alors isolé de l'interface 7 et du au moins un analyseur de gaz 5, 5b. La vanne trois voies 8 est d'autre part, configurée pour mettre en communication ledit au moins un analyseur de gaz 5, 5b avec le générateur d'humidité 9, l'interface 7 étant alors isolée du générateur d'humidité 9 et du au moins un analyseur de gaz 5, 5b. Alternativement, on peut prévoir par exemple une vanne distincte dans chaque dérivation de la canalisation de mesure 6 pour assurer la même fonction.

Selon un exemple de réalisation, l'analyseur de gaz 5, 5b comporte une petite pompe de prélèvement, dont le débit de prélèvement est inférieur à 5 SLM (pour « Standard Litres Per Minute » en anglais), tel que de l'ordre de 1.2 SLM.

Ainsi, selon un premier exemple de mise en œuvre du procédé de mesure, le flux de gaz contenant de la vapeur d'eau peut être dirigé par prélèvement de la pompe, du générateur d'humidité 9 vers l'analyseur de gaz 5, 5b, l'interface 7 étant alors isolée du générateur d'humidité 9 et de l'analyseur de gaz 5, 5b.

De même, les espèces gazeuses à mesurer peuvent être dirigées par prélèvement de la pompe, de la boîte de transport 2 vers l'analyseur de gaz 5, 5b, le générateur d'humidité 9 étant alors isolé de l'interface 7 et de l'analyseur de gaz 5, 5b.

L'analyseur de gaz 5, 5b permet de mesurer la concentration d'au moins une espèce gazeuse en temps réel, c'est-à-dire avec une durée de mesure inférieure à quelques secondes, voire quelques minutes, pour de faibles concentrations de l'ordre du ppm ou du ppb.

Selon un exemple de réalisation, l'analyseur de gaz 5, 5b comporte un capteur optique fonctionnant sur le principe de spectroscopie d'absorption de la longueur d'onde d'un laser par une espèce gazeuse à mesurer, tel que par exemple le principe dit CRDS, pour « Cavity Ring-Down Spectroscopy » en anglais. Pour cela, l'analyseur de gaz 5, 5b comporte une cavité optique en contact avec l'espèce gazeuse à mesurer et un laser configuré pour illuminer la cavité optique avec une longueur d'onde prédéfinie.

Lorsqu'une espèce gazeuse traverse la cavité optique, celle-ci absorbe la lumière qui diminue corrélativement. On mesure le temps de décroissance de l'intensité initiale de la lumière, et ce temps peut être utilisé pour calculer la concentration de la substance absorbante dans le mélange de gaz présent dans la cavité optique.

En utilisation, on commence par orienter la vanne trois voies 8 de manière à mettre en communication les gaz provenant d'une boîte de transport 2 positionnée sur l'interface 7, avec au moins un analyseur de gaz 5, 5b. Le générateur d'humidité 9 est alors isolé de l'interface 7 et du au moins un analyseur de gaz 5, 5b.

On mesure la concentration d'au moins une espèce gazeuse, potentiellement présente à l'intérieur de la boîte de transport 2 et potentiellement polluante, par au moins un analyseur de gaz 5, 5b.

L'espèce gazeuse mesurée est par exemple un acide, comme l'acide fluorhydrique HF ou l'acide chlorhydrique HCl ou un solvant, tel que le PGMEA (propylène glycol methyl ether).

Selon un autre exemple, l'espèce gazeuse est l'ammoniaque NH₃.

Dans le cas où le dispositif de mesure comporte plusieurs analyseurs de gaz 5, 5b, on peut prévoir que chaque analyseur de gaz 5, 5b soit adapté pour la mesure d'une espèce gazeuse distincte ou d'un groupe d'espèces gazeuses distinctes.

Ainsi, par exemple, un premier analyseur de gaz 5 est adapté pour mesurer l'acide fluorhydrique HF, un deuxième analyseur de gaz 5b est adapté pour mesurer l'acide chlorhydrique HCl et un troisième analyseur de gaz (non représenté) est adapté pour mesurer l'ammoniaque NH₃.

Dans l'exemple illustré sur la figure 3, l'espèce gazeuse mesurée est l'acide fluorhydrique HF.

La mesure est effectuée pendant une durée par exemple de l'ordre de deux minutes. Pendant cette durée, si de l'acide fluorhydrique HF est présent dans l'atmosphère intérieure de la boîte de transport 2, une proportion de l'acide fluorhydrique HF transitera jusque dans le volume de la cellule d'analyse, alors que la proportion restante sera adsorbée sur les parois internes de la canalisation de mesure 6 et de l'analyseur de gaz 5.

Après la mesure, on met en communication l'analyseur de gaz 5 avec le générateur d'humidité 9 et on fournit un flux de gaz contenant de la vapeur d'eau dans le dispositif de mesure. L'interface 7 est alors isolée du générateur d'humidité 9 et de l'analyseur de gaz 5.

Le flux de gaz contenant de la vapeur d'eau est introduit dans le dispositif de mesure, c'est-à-dire dans la portion de la canalisation de mesure 6 reliant le générateur d'humidité 9 à l'analyseur de gaz 5. Le flux de gaz contenant de la vapeur d'eau s'écoule ainsi du générateur d'humidité 9 vers l'analyseur de gaz 5 à travers la canalisation de mesure 6. Le flux de gaz contenant de la vapeur d'eau peut aussi être introduit directement dans l'analyseur de gaz 5 du dispositif de mesure.

Le flux de gaz contenant de la vapeur d'eau est un mélange gazeux ayant un taux d'humidité supérieur à 0% à température ambiante (20°C), par exemple supérieur à 20% d'humidité.

On prévoit par exemple que le flux de gaz présente un taux d'humidité supérieur à 40%, tel que supérieur à 95%. Il est ainsi supérieur au taux d'humidité de la salle blanche généralement proche de 40%.

Le flux de gaz contenant de la vapeur d'eau est par exemple de l'air humide formé par exemple à partir d'un mélange d'air sec de type CDA (pour « Compressed Dry Air » en anglais) et de vapeur d'eau de manière à mieux contrôler le niveau d'humidité. Selon un autre exemple, le flux de gaz contenant de la vapeur d'eau est un mélange d'azote et de vapeur d'eau.

L'introduction du flux de gaz contenant de la vapeur d'eau dans le dispositif de mesure peut être réalisée lorsqu'une mesure de la concentration d'acide fluorhydrique HF est supérieure à un seuil prédéterminé.

Alternativement, l'introduction du flux de gaz contenant de la vapeur d'eau dans le dispositif de mesure est réalisée après chaque mesure de la concentration d'acide fluorhydrique HF. On peut prévoir en outre de fournir un flux de gaz présentant un premier taux d'humidité si la concentration d'acide fluorhydrique HF est inférieure à un seuil prédéterminé et de fournir un flux de gaz présentant un deuxième taux d'humidité supérieur au premier taux d'humidité si la concentration d'acide fluorhydrique HF dépasse le seuil prédéterminé.

La figure 3 représente la quantité d'acide fluorhydrique HF désorbée en fonction du temps dans un dispositif de mesure en fonction de différents taux d'humidité de l'air introduit.

La canalisation de mesure 6 et l'analyseur de gaz 5 sont préalablement pollués de manière à présenter une même quantité d'acide fluorhydrique HF absorbée. Des flux d'air contenant différents taux d'humidité provenant du générateur d'humidité 9, sont ensuite introduits dans la canalisation de mesure 6, et correspondent aux différentes courbes décrites ci-après.

La courbe A correspond à la quantité d'acide fluorhydrique HF désorbée par la canalisation de mesure 6 et l'analyseur de gaz 5 suite à l'introduction d'un air sec, la courbe B correspond à la quantité d'acide fluorhydrique HF désorbée suite à l'introduction d'un air présentant un taux d'humidité de l'ordre de 32% et la courbe C correspond à la quantité d'acide fluorhydrique HF désorbée suite à l'introduction d'un air à 63% d'humidité.

On constate sur ce graphique que, plus le taux d'humidité de l'air est élevé et plus la quantité d'acide fluorhydrique HF désorbée augmente (courbe B et C).

On constate également que la quantité d'acide fluorhydrique HF désorbée est quasiment nulle pour une injection d'air sec dans le dispositif de mesure (courbe A).

Ainsi, le taux d'humidité du flux gazeux introduit pour dépolluer le dispositif de mesure, influence la capacité de l'acide fluorhydrique HF à désorber des parois.

Suite à l'introduction d'un flux de gaz sec (courbe A), l'acide fluorhydrique HF reste absorbé sur les parois. En revanche, l'apport l'humidité dans la canalisation de mesure 6 ainsi que dans l'analyseur de gaz 5 permet d'accélérer la désorption de l'acide fluorhydrique HF des parois.

Il est donc possible d'accélérer la dépollution du dispositif de mesure entre deux mesures successives par l'introduction d'un flux de gaz humide dans le dispositif de mesure et donc de diminuer le temps d'attente entre deux mesures.

Selon un deuxième exemple de mise en oeuvre du procédé de mesure, on fournit un flux de gaz contenant de la vapeur d'eau dans le dispositif de mesure pendant que l'on réalise une mesure de contamination.

Pour cela, le générateur d'humidité 9 est mis en communication à la fois avec l'interface 7 et le au moins un analyseur de gaz 5, 5b. Le flux de gaz contenant de la vapeur d'eau est introduit dans la canalisation de mesure 6 reliant le au moins un analyseur de gaz 5, 5b à l'interface 7 pendant que le au moins un analyseur de gaz 5, 5b effectue une mesure de contamination.

On prévoit en outre que le flux de gaz contenant de la vapeur d'eau soit inférieur au flux prélevé par la pompe du au moins un analyseur de gaz 5, 5b pour éviter que le flux de gaz contenant de la vapeur d'eau puisse « remonter » dans la boite de transport 2.

On peut retrouver la concentration de l'espèce gazeuse diluée par le flux de gaz contenant de la vapeur d'eau en appliquant un facteur correctif proportionnel. Par exemple, on multiplie par deux le résultat de mesure si une moitié du flux de gaz mesuré provient de la boite de transport 2 et l'autre moitié provient du générateur d'humidité 9.

Ce procédé de mesure est particulièrement avantageux pour la mesure de la contamination de boîtes de transport 2 purgées sous atmosphère sèche. En effet, le gaz de purge sec de la boîte de transport 2 qui est prélevé par l'analyseur de gaz 5, 5b, favorise l'absorption de l'acide fluorhydrique sur les parois. En fournissant de l'air humide dans le dispositif de mesure pendant la mesure, on facilite le transport de l'acide fluorhydrique jusqu'à l'analyseur de gaz 5, 5b ce qui permet de mieux voir les espèces gazeuses présentes dans la boîte de transport 2.

## Revendications

1. Procédé de mesure de la contamination d'une boîte de transport (2) pour le convoyage et le stockage atmosphérique de substrats (3) dans lequel on mesure la concentration d'au moins une espèce gazeuse à l'intérieur de la boîte de transport (2) par un dispositif de mesure comprenant au moins un analyseur de gaz (5, 5b) et une canalisation de mesure (6) reliant ledit au moins un analyseur de gaz (5, 5b) à une interface (7), l'interface (7) mettant en communication la canalisation de mesure (6) avec l'atmosphère interne d'au moins une boîte de transport (2), **caractérisé en ce qu'**on fournit un flux de gaz contenant de la vapeur d'eau dans le dispositif de mesure.

2. Procédé de mesure selon la revendication précédente, dans lequel au moins une espèce gazeuse dont on mesure la concentration est l'acide fluorhydrique (HF).

3. Procédé de mesure selon l'une des revendications précédentes, dans lequel le flux de gaz contenant de la vapeur d'eau présente un taux d'humidité supérieur à 40%.

4. Procédé de mesure selon l'une des revendications précédentes, dans lequel le flux de gaz contenant de la vapeur d'eau présente un taux d'humidité supérieur à 95%.

5. Procédé de mesure selon l'une des revendications précédentes, dans lequel le flux de gaz contenant de la vapeur d'eau est de l'air humide.

6. Procédé de mesure selon l'une des revendications précédentes, dans lequel on fournit un flux de gaz contenant de la vapeur d'eau dans le dispositif de mesure pendant que l'on réalise une mesure de contamination.

7. Procédé de mesure selon l'une des revendications 1 à 5, dans lequel on fournit un flux de gaz contenant de la vapeur d'eau dans le dispositif de mesure après avoir réalisé une mesure de contamination et isolé l'interface (7).

8. Procédé de mesure selon la revendication précédente, dans lequel on fournit un flux de gaz contenant de la vapeur d'eau dans le dispositif de mesure après avoir réalisé une mesure de contamination pour laquelle la concentration de l'espèce gazeuse a dépassé un seuil prédéterminé.

9. Procédé de mesure selon la revendication 7, dans lequel on fournit un flux de gaz contenant de la vapeur d'eau dans le dispositif de mesure après chaque mesure d'espèce gazeuse.

10. Procédé de mesure selon la revendication précédente, dans lequel on fournit un flux de gaz présentant un premier taux d'humidité si la concentration de l'espèce gazeuse est inférieure à un seuil prédéterminé et on fournit un flux de gaz présentant un deuxième taux d'humidité supérieur au premier taux d'humidité si la concentration de l'espèce gazeuse dépasse le seuil prédéterminé.

11. Station de mesure de la contamination d'une boîte de transport pour le convoyage et le stockage atmosphérique de substrats comprenant :
- un dispositif de mesure comprenant au moins un analyseur de gaz (5, 5b) et une canalisation de mesure (6) reliée audit au moins un analyseur de gaz (5, 5b), et
- une interface (7) pour mettre en communication la canalisation de mesure (6) avec l'atmosphère interne d'au moins une boîte de transport (2) de manière à effectuer une analyse d'au moins une espèce gazeuse contenue dans l'atmosphère interne de la boîte de transport (2) par ledit au moins un analyseur de gaz (5, 5b),
**caractérisée en ce qu'**elle comporte un générateur d'humidité (9) configuré pour fournir un flux de gaz contenant de la vapeur d'eau dans le dispositif de mesure.

12. Station de mesure selon la revendication précédente, **caractérisée en ce que** le générateur d'humidité (9) est raccordé à la canalisation de mesure (6) reliant le au moins un analyseur de gaz (5, 5b) à l'interface (7), la station de mesure comportant au moins une vanne (8 ; 8a, 8b, 8c) agencée sur la canalisation de mesure (6), la vanne (8 ; 8a, 8b, 8c) étant configurée pour mettre en communication l'interface (7) avec le au moins un analyseur de gaz (5, 5b) ou le au moins un analyseur de gaz (5, 5b) avec le générateur d'humidité (9).

13. Station de mesure selon l'une des revendications 11 ou 12, **caractérisée en ce que** le au moins un analyseur de gaz (5, 5b) comporte une pompe de prélèvement.

14. Station de mesure selon l'une des revendications 11 à 13, **caractérisée en ce que** le au moins un analyseur de gaz (5, 5b) comporte un capteur optique fonctionnant par spectroscopie d'absorption.

## Patentansprüche

1. Verfahren zur Messung der Kontamination einer Transportkiste (2) für den Transport und die Lagerung unter Atmosphäre von Substraten (3), wobei man die Konzentration mindestens einer Gasspezies im Inneren der Transportkiste (2) durch eine Messvorrichtung misst, die mindestens einen Gasanalysator (5, 5b) und eine Messleitung (6), die den mindestens einen Gasanalysator (5, 5b) mit einer Grenzfläche (7) verbindet, umfasst, wobei die Grenzfläche (7) die Messleitung (6) mit der Innenatmosphäre mindestens einer Transportkiste (2) in Kommunikation bringt, **dadurch gekennzeichnet, dass** man der Messvorrichtung einen wasserdampfhaltigen Gasstrom zuführt.

2. Messverfahren nach dem vorhergehenden Anspruch, wobei mindestens eine Gasspezies, deren Konzentration gemessen wird, Flusssäure (HF) ist.

3. Messverfahren nach dem vorhergehenden Anspruch, wobei der wasserdampfhaltige Gasstrom einen Feuchtigkeitsanteil von mehr als 40 % aufweist.

4. Messverfahren nach einem der vorhergehenden Ansprüche, wobei der wasserdampfhaltige Gasstrom einen Feuchtigkeitsanteil von mehr als 95 % aufweist.

5. Messverfahren nach einem der vorhergehenden Ansprüche, wobei es sich bei dem wasserdampfhaltigen Gasstrom um feuchte Luft handelt.

6. Messverfahren nach einem der vorhergehenden Ansprüche, wobei man der Messvorrichtung einen wasserdampfhaltigen Gasstrom zuführt, während man eine Kontaminationsmessung durchführt.

7. Messverfahren nach einem der Ansprüche 1 bis 5, wobei man der Messvorrichtung einen wasserdampfhaltigen Gasstrom zuführt, nachdem man eine Kontaminationsmessung durchgeführt und die Grenzfläche (7) isoliert hat.

8. Messverfahren nach dem vorhergehenden Anspruch, wobei man der Messvorrichtung einen wasserdampfhaltigen Gasstrom zuführt, nachdem man eine Kontaminationsmessung durchgeführt hat, bei der die Konzentration der Gasspezies einen zuvor festgelegten Schwellenwert überschritten hat.

9. Messverfahren nach Anspruch 7, wobei man der Messvorrichtung einen wasserdampfhaltigen Gasstrom nach jeder Messung der Gasspezies zuführt.

10. Messverfahren nach dem vorhergehenden Anspruch, wobei man einen Gasstrom zuführt, der einen ersten Feuchtigkeitsanteil aufweist, wenn die Konzentration der Gasspezies kleiner als ein zuvor festgelegter Schwellenwert ist, und man einen Gasstrom zuführt, der einen zweiten Feuchtigkeitsanteil aufweist, der höher ist als der erste Feuchtigkeitsanteil, wenn die Konzentration der Gasspezies den zuvor festgelegten Schwellenwert überschreitet.

11. Station zum Messen der Kontamination einer Transportkiste für den Transport und die Lagerung unter Atmosphäre von Substraten, umfassend:
- eine Messvorrichtung, die mindestens einen Gasanalysator (5, 5b) und eine Messleitung (6) umfasst, die an dem mindestens einen Gasanalysator (5, 5b) angeschlossen ist, und
- eine Grenzfläche (7) zum Herstellen einer Kommunikation der Messleitung (6) mit der Innenatmosphäre mindestens einer Transportkiste (2), um eine Analyse mindestens einer in der Innenatmosphäre der Transportkiste (2) enthaltenen Gasspezies durch den mindestens einen Gasanalysator (5, 5b) durchzuführen,
**dadurch gekennzeichnet, dass** sie einen Feuchtigkeitsgenerator (9) umfasst, der dafür ausgelegt ist, der Messvorrichtung einen wasserdampfhaltigen Gasstrom zuzuführen.

12. Messstation nach dem vorhergehenden Anspruch, **dadurch gekennzeichnet, dass** der Feuchtigkeitsgenerator (9) mit der Messleitung (6) verbunden ist, die den mindestens einen Gasanalysator (5, 5b) mit der Grenzfläche (7) verbindet, wobei die Messstation mindestens ein an der Messleitung (6) angebrachtes Ventil (8; 8a, 8b, 8c) umfasst, wobei das Ventil (8; 8a, 8b, 8c) dafür ausgelegt ist, die Grenzfläche (7) mit dem mindestens einen Gasanalysator (5, 5b) in Kommunikation zu bringen oder den mindestens einen Gasanalysator (5, 5b) mit dem Feuchtigkeitsgenerator (9) in Kommunikation zu bringen.

13. Messstation nach einem der Ansprüche 11 oder 12, **dadurch gekennzeichnet, dass** der mindestens eine Gasanalysator (5, 5b) eine Probennahmepumpe umfasst.

14. Messstation nach einem der Ansprüche 11 bis 13, **dadurch gekennzeichnet, dass** der mindestens eine Gasanalysator (5, 5b) einen optischen Sensor umfasst, der mittels Absorptionsspektroskopie arbeitet.

## Claims

1. Method for measuring the contamination of a transport box (2) for the atmospheric shipping and storage of substrates (3) in which the concentration of at least one gaseous species inside the transport box (2) is measured by a measurement device comprising at least one gas analyser (5, 5b) and a measurement line (6) connecting said at least one gas analyser (5, 5b) to an interface (7), the interface (7) placing the measurement line (6) in communication with the internal atmosphere of at least one transport box (2), **characterized in that** a gas flow containing water vapour is supplied to the measurement device.

2. Measurement method according to the preceding claim, in which at least one gaseous species, the concentration of which is measured, is hydrofluoric acid (HF).

3. Measurement method according to either of the preceding claims, in which the gas flow containing water vapour has a degree of humidity of greater than 40%.

4. Measurement method according to one of the preceding claims, in which the gas flow containing water vapour has a degree of humidity of greater than 95%.

5. Measurement method according to one of the preceding claims, in which the gas flow containing water vapour is moist air.

6. Measurement method according to one of the preceding claims, in which a gas flow containing water vapour is supplied to the measurement device while a contamination measurement is carried out.

7. Measurement method according to one of Claims 1 to 5, in which a gas flow containing water vapour is supplied to the measurement device after having carried out a contamination measurement and isolated the interface (7).

8. Measurement method according to the preceding claim, in which a gas flow containing water vapour is supplied to the measurement device after having carried out a contamination measurement for which the concentration of the gaseous species has exceeded a predetermined threshold.

9. Measurement method according to Claim 7, in which a gas flow containing water vapour is supplied to the measurement device after each gaseous species measurement.

10. Measurement method according to the preceding claim, in which a gas flow having a first degree of humidity is supplied if the concentration of the gaseous species is lower than a predetermined threshold and a gas flow having a second degree of humidity greater than the first degree of humidity is supplied if the concentration of the gaseous species exceeds the predetermined threshold.

11. Station for measuring the contamination of a transport box for the atmospheric shipping and storage of substrates, comprising:
- a measurement device comprising at least one gas analyser (5, 5b) and a measurement line (6) connected to said at least one gas analyser (5, 5b), and
- an interface (7) for placing the measurement line (6) in communication with the internal atmosphere of at least one transport box (2) so as to carry out an analysis of at least one gaseous species contained in the internal atmosphere of the transport box (2) by said at least one gas analyser (5, 5b),
**characterized in that** it comprises a humidity generator (9) configured in order to supply a gas flow containing water vapour to the measurement device.

12. Measurement station according to the preceding claim, **characterized in that** the humidity generator (9) is coupled to the measurement line (6) connecting the at least one gas analyser (5, 5b) to the interface (7), the measurement station comprising at least one valve (8; 8a, 8b, 8c) arranged on the measurement line (6), the valve (8; 8a, 8b, 8c) being configured in order to place the interface (7) in communication with the at least one gas analyser (5, 5b) or to place the at least one gas analyser (5, 5b) in communication with the humidity generator (9).

13. Measurement station according to either of Claims 11 and 12, **characterized in that** the at least one gas analyser (5, 5b) comprises a sampling pump.

14. Measurement station according to one of Claims 11 to 13, **characterized in that** the at least one gas analyser (5, 5b) comprises an optical sensor that operates by absorption spectroscopy.
